# EUROPEAN PATENT SPECIFICATION

(11) **EP 0 690 838 B1**
(45) Date of publication and mention of the grant of the patent: **10.09.1997**
(21) Application number: 94910372.5
(22) Date of filing: 07.03.1994
(51) Int. Cl.: C07C 303/06, C07C 309/31

(54) **PROCESS TO MANUFACTURE ALKYLBENZENESULPHONIC ACID**
VERFAHREN ZUR HERSTELLUNG VON ALKYLBENZOLSULFONSÄURE
PROCEDE POUR LA PREPARATION DE L'ACIDE BENZENE-SULFONIQUE

(30) Priority: 11.03.1993 GB 9305173
(43) Date of publication of application: 10.01.1996
(73) Proprietor: UNILEVER PLC, London EC4P 4BQ (GB); UNILEVER N.V., 3013 AL Rotterdam (NL)
(72) Inventor: ADAMS, Amanda, Jane, Merseyside L63 2LF (GB); ROBERTS, David, William, Merseyside L63 9LD (GB)
(74) Representative: Elliott, Peter William
(86) International application number: EP9400665
(87) International publication number: WO9420458

(56) References cited:
- GB-A- 669 899
- US-A- 4 113 765

## Description

This invention relates to the production of alkyl benzene sulphonic acid having the general formula 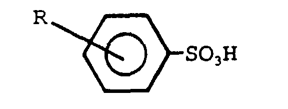 where R denotes an alkyl group of six to 20 carbon atoms.

The soluble salts of alkyl benzene sulphonic acid have been commercially very important as anionic detergents for many years. The customary method of production starts with the appropriate alkyl benzene which is sulphonated and then subsequently neutralised. The sulphonation reaction can be carried out in various ways. In the past, the reaction has been carried out using concentrated sulphuric acid or oleum, in either case using an excess of acid. At the end of the sulphonation the surplus acid and the desired product must be separated. Alternatively the entire mixture can be neutralised leading to a product containing a substantial proportion of an inorganic sulphate salt, e.g. sodium sulphate.

Subsequently, it has become conventional to use sulphur trioxide as sulphonating agent. US patent 2928867 published in 1960 teaches that at the end of a sulphonation process carried out with sulphur trioxide, a small amount of water should be added to the reaction mixture to hydrolyse sulphonic acid anhydrides present in the reaction mixture so that a stable product is obtained after neutralisation.

During more recent years it has become standard practice to use gaseous sulphur trioxide as sulphonating agent in an approximately stoichiometric quantity, then age the resulting mixture during which time the reaction mixture proceeds further towards completion, and next add a small quantity of water which is believed to hydrolyse any anhydrides present in the reaction mixture thereby stabilising reaction. This current practice is described in, for example, "Sulphonation Technology in the Detergent Industry" by W Herman de Groot 1991 especially pages 8 and 9.

When carrying out this conventional practice the sulphur trioxide is normally diluted with some other gas which does not react. Air can be used for this.

It is possible to carry out the reaction using an excess of sulphur trioxide but this leads to disadvantages. One disadvantage is the formation of coloured impurities. If a substantial excess of sulphur trioxide is used, the mixture obtained contains unwanted sulphuric acid which must be removed or tolerated as an impurity just as with the older processes using sulphuric acid or oleum. This is less of a problem if there is only a small excess of sulphur trioxide but, as will be explained below, this leads to the presence of undesirable impurities.

When sulphonation is carried out using an approximately stoichiometric quantity of sulphur trioxide it is found that the conventional ageing period at the end of the sulphonation reaction gives increased conversion of the alkyl benzene into the desired sulphonic acid. Nevertheless, it is observed that the alkyl benzene feedstock is not completely converted into the desired alkyl benzene sulphonic acid. A small percentage of alkyl benzene remains unchanged and a small quantity is converted into a sulphone of the formula

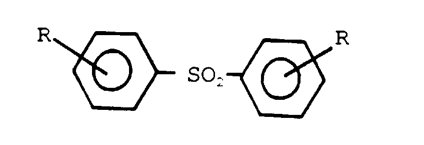

The relative proportions of unconverted alkyl benzene and sulphone impurity vary. They are affected by the reaction conditions employed and by the mole ratio of sulphur trioxide to alkyl benzene. Although it is possible to vary the relative proportions of unreacted alkyl benzene and sulphone it is not possible to avoid both of them simultaneously by control of reaction conditions or mole ratio of reactants. Thus if the mole ratio of sulphur trioxide to alkyl benzene is increased so as to minimise the percentage of unconverted alkyl benzene, the percentage of sulphone increases.

Failure to convert alkyl benzene to sulphonic acid and conversion to by-product sulphone both represent a waste of alkyl benzene feedstock. Additionally, unconverted alkyl benzene can give rise to tower emission problems on spray drying and there have also been suggestions that residual alkyl benzene is slightly toxic.

Thus, although the present commercial procedure of sulphonation with stoichiometric quantity of sulphur trioxide followed by ageing and then the addition of water to arrest the reaction is very advantageous compared with earlier processes in that it gives a high level of conversion to the desired product without introducing large quantities of sulphuric acid into the product mixture, there is still some scope for further improvement in that it would be desirable to reduce further the combined levels of alkyl benzene and sulphone below what is currently achievable.

According to the present invention there is provided a method of manufacturing alkyl benzene sulphonate by contacting, as reactants, alkyl benzene with sulphur trioxide using a molar ratio of alkyl benzene to sulphur trioxide in a range from 1:0.9 to 1:1.3, characterised by incorporating sulphuric acid into the reaction mixture after bringing the reactants into contact, the amount of sulphuric acid being not more than 10% by weight of the reaction mixture, and then allowing reaction in the mixture to continue for at least 30 minutes.

We have found that by incorporating sulphuric acid in this manner it is possible to achieve an improvement in the overall conversion to the desired product and a consequent reduction in the undesired impurities.

For carrying out this reaction the sulphur trioxide is preferably diluted with another gas, as is conventional. This other gas may well be air. The ratio of sulphur trioxide to other gas may desirably lie in a range from 3% to 10% by volume.

The molar ratio of alkyl benzene to sulphur trioxide is preferably in a range from 1:0.9 to 1:1. better 1:0.95 to 1:1.

The alkyl benzene which is sulphonated may have a straight or branched alkyl group which preferably contains from 10 to 15 carbon atoms. Although branched alkyl benzene has been extensively used for detergent manufacture it is nowadays usually preferred to employ a linear alkyl benzene, that is to say alkyl benzene in which the alkyl group has a straight carbon chain.

It is known to carry out the sulphonation of alkyl benzene which is provided as a mixed feedstock also containing some other material which undergoes sulphonation or sulphation. Conceivably this invention could be employed when it is a mixed feedstock which undergoes reaction. However the invention is particularly significant when the alkyl benzene feedstock is not mixed with a substantial quantity of any other material so that the feedstock contains in excess of 90% by weight alkyl benzene.

The sulphuric acid which is added may be in the form of conventional concentrated sulphuric acid containing 98% of sulphuric acid itself. It would also be possible, although it is not preferred, to employ sulphuric acid which is not quite so concentrated. Alternatively it would be possible to employ fuming sulphuric acid containing some oleum.

The amount of sulphuric acid which is employed is preferably at least 1% and more preferably at least 1.5% by weight of the reaction mixture. The amount is preferably not more than 5% of the weight of the reaction mixture and quite possibly not more than 4% by weight of the reaction mixture.

Ageing of the reaction mixture after addition of the sulphuric acid preferably takes place with some agitation of the reaction mixture and at a temperature somewhat above normal room temperature, e.g. in the range from 30 to 70°C. The ageing period will generally not exceed 12 hours although longer periods may be employed if desired. An ageing period in the range from 2 to 8 hours is preferred. At the end of this ageing period some water may be added to the reaction mixture to hydrolyse any remaining sulphur trioxide and stabilise the reaction mixture, in accordance with conventional practice for sulphonation.

The invention will be further explained and illustrated by the following example in which all amounts and proportions are by weight unless otherwise stated.

Alkyl benzene having a molecular weight of approximately 218 corresponding to a formula 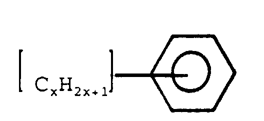 in which the average value of x is 10 was sulphonated in a commercial sulphonation plant with a multi-tube falling film reactor. Sulphonation was effected using sulphur trioxide and air in an approximate ratio of 1.00.

Quantities of reaction mixture leaving the falling film reactor were taken, mixed with varying amounts of 98% sulphuric acid, and then aged at 50°C for up to 4 hours. Samples were taken out of the mixtures after various intervals of time and neutralised to the sodium salt of the acid under conditions calculated to give approximately 25% sodium alkyl benzene sulphonate in the neutralised mixture. These neutralised mixtures were analysed to determine the actual content of sulphonic acid and also to determine the content of non-detergent organic matter (NDOM). This NDOM consists principally of unreacted alkyl benzene (LAB) and of sulphone by-product. The amounts of these materials were determined from the infra-red spectrum of the NDOM.

The results obtained are set out in the following Table 1 in which the amounts of sulphone, unreacted alkyl benzene and overall non-detergent organic matter are quoted as percentages by weight of the amount of alkyl benzene sulphonic acid (as determined by analysis).

The results obtained are also shown in a different way in Table 2 below. In this Table the amounts of alkyl benzene sulphonic acid, sulphone impurity and unreacted alkyl benzene in the reaction mixture are expressed as mole percentages of the alkyl benzene feedstock.

**Table 1**

| Impurities as % by weight of sulphonic acid | | | | |
|---|---|---|---|---|
| % added H₂SO₄ | Ageing Time (hr) | Sulphone (%) | LAB (%) | NDOM (%) |
| | 0 | 0.8 | 3.7 | 4.7 |
| | 1 | 0.8 | 1.0 | 2.1 |
| | 2 | 1.1 | 1.4 | 2.6 |
| None | 3 | 0.9 | 1.2 | 2.2 |
| | 4 | 0.9 | 1.2 | 2.3 |
| | 24 | - | - | - |
| | | | | |
| | 1 | 0.6-1.1 | 0.5-0.8 | 1.2-2.0 |
| | 2 | 0.7 | 0.3 | 1.2 |
| 2 | 3 | 0.6 | 0.2 | 0.8 |
| | 4 | 0.3 | 0.1 | 0.4 |
| | | | | |
| | 1 | 0.5-0.8 | 0.1-0.2 | 0.8-1.2 |
| | 2 | 0.5 | 0.2 | 0.8 |
| 3 | 3 | 0.7 | 0.4 | 1.3 |
| | 4 | 0.8 | 0.3 | 1.3 |

**Table 2**

| Fate of the initial LAB feedstock, molar % basis | | | | |
|---|---|---|---|---|
| | | Converted to: | | |
| % added H₂SO₄ | Ageing Time (hr) | Sulphonic acid | Sulphone | Remaining as LAB |
| | 0 | 94.5 | 0.9 | 4.7 |
| | 1 | 97.7 | 0.9 | 1.4 |
| | 2 | 97.0 | 1.2 | 1.8 |
| None | 3 | 97.5 | 1.0 | 1.5 |
| | 4 | 97.4 | 1.1 | 1.5 |
| | 24 | - | - | - |
| | | | | |
| | 1 | 98.6-97.7 | 0.8-1.2 | 0.6-1.0 |
| | 2 | 98.8 | 0.8 | 0.4 |
| 2 | 3 | 99.1 | 0.7 | 0.2 |
| | 4 | 99.6 | 0.3 | 0.1 |
| | | | | |
| | 1 | 98.9-99.2 | 0.9-0.6 | 0.3-0.2 |
| | 2 | 99.2 | 0.6 | 0.2 |
| 3 | 3 | 98.7 | 0.8 | 0.5 |
| | 4 | 98.6 | 1.0 | 0.4 |

It can be seen from these results that without addition of sulphuric acid there is substantial conversion of the feedstock to the desired product before ageing, and this conversion rises to a higher level during the ageing period. However, when sulphuric acid is added in an amount of 2% or 3% by weight the extent of conversion to the desired sulphonic acid is even greater after ageing for 1 hour or more, the quantity of sulphone impurity is unchanged or slightly reduced and the quantity of unreacted alkyl benzene is significantly reduced.

This experiment was repeated during a subsequent production run on the same commercial plant. Sulphuric acid was added in amounts of 2, 3 and 4% by weight of the reaction mixture. The results obtained are set out in Tables 3 and 4 below. Again, the addition of 2, 3 or 4% by weight of sulphuric acid to the reaction mixture led in each case to a reduction in the amount of non-detergent organic matter with only a small content of unreacted alkyl benzene.

**Table 3**

| Impurities as % by weight of sulphonic acid | | | | |
|---|---|---|---|---|
| % added H₂SO₄ | Ageing Time (hr) | Sulphone (%) | LAB (%) | NDOM (%) |
| | 0 | - | - | 7.2 |
| | 1 | - | - | 4.5 |
| | 2 | - | - | 2.1 |
| None | 3 | - | - | 2.5 |
| | 4 | - | - | 2.6 |
| | 24 | - | - | 2.5 |
| | | | | |
| | 1 | 0.9 | 0.2 | 1.5 |
| | 2 | 0.7 | <0.1 | 1.1 |
| 2 | 3 | 0.9 | 0.1 | 1.3 |
| | 4 | 0.9 | 0.2 | 1.4 |
| | | | | |
| | 1 | 1.3 | 0.7 | 2.7 |
| | 2 | 0.7 | 0.4 | 1.5 |
| 3 | 3 | 0.9 | 0.5 | 1.8 |
| | 4 | 0.7 | <0.1 | 1.2 |
| | | | | |
| | 1 | 0.9 | 0.6 | 2.0 |
| | 2 | 0.8 | 0.5 | 1.7 |
| 4 | 3 | 0.9 | 0.5 | 1.8 |
| | 4 | 0.6 | 0.3 | 1.2 |

**Table 4**

| Fate of the initial LAB feedstock, molar % basis | | | | |
|---|---|---|---|---|
| | | Converted to: | | |
| % added H₂SO₄ | Ageing Time (hr) | Sulphonic acid | Sulphone | Remaining as LAB |
| | 0 | 91.6 | | |
| | 1 | 94.6 | | |
| | 2 | 97.3 | | |
| None | 3 | 96.9 | | |
| | 4 | 96.8 | | |
| | 24 | 96.9 | | |
| | | | | |
| | 1 | 98.7 | 1.0 | 0.3 |
| | 2 | 99.2 | 0.8 | <0.1 |
| 2 | 3 | 98.9 | 1.0 | 0.1 |
| | 4 | 98.8 | 1.0 | 0.2 |
| | | | | |
| | 1 | 97.5 | 1.5 | 1.0 |
| | 2 | 98.6 | 0.9 | 0.5 |
| 3 | 3 | 98.2 | 1.1 | 0.7 |
| | 4 | 99.1 | 0.85 | 0.05 |
| | | | | |
| | 1 | 98.1 | 1.1 | 0.8 |
| | 2 | 98.4 | 1.0 | 0.6 |
| 4 | 3 | 98.3 | 1.0 | 0.7 |
| | 4 | 97.9 | 1.2 | 0.9 |

## Claims

1. A method of manufacturing alkyl benzene sulphonate by contacting, as reactants, alkyl benzene with sulphur trioxide using a molar ratio of alkyl benzene to sulphur trioxide in the range from 1:0.9 to 1:1.3, characterized by incorporating sulphuric acid into the reaction mixture after bringing the reactants into contact with each other, the amount of sulphuric acid being not more than 10% of the reaction mixture, and then allowing reaction in the mixture to continue for at least 15 minutes.

2. A method according to claim 1 wherein the sulphur trioxide is diluted with another gas.

3. A method according to claim 1 or claim 2 wherein the molar ratio of alkyl benzene to sulphur trioxide lies in a range from 1:0.95 to 1:1.1.

4. A method according to claim 1, claim 2 or claim 3 wherein the amount of sulphuric acid lies in a range from 1 to 5% by weight of the reaction mixture.

5. A method according to any one of the preceding claims wherein, after incorporation of the sulphuric acid, reaction is allowed to continue for a period of time lying in a range from 30 minutes to 8 hours.

6. A method according to any one of the preceding claims wherein the mixture is maintained at a temperature in the range 30 to 70°C after incorporation of the sulphuric acid therein.

## Patentansprüche

1. Ein Verfahren zur Herstellung von Alkylbenzolsulfonat durch In-Kontakt-bringen, als Reaktionsteilnehmer, von Alkylbenzol mit Schwefeltrioxid unter Verwendung eines molaren Verhältnisses von Alkylbenzol zu Schwefeltrioxid in dem Bereich von 1 : 0,9 bis 1 : 1,3, gekennzeichnet durch Inkorporieren von Schwefelsäure in die Reaktionsmischung nach dem In-Kontakt-bringen der Reaktionsteilnehmer miteinander, wobei die Menge der Schwefelsäure nicht größer als 10 Gewichtsprozent der Reaktionsmischung beträgt, und anschließendem Ermöglichen der Reaktion in der Mischung, sich für zumindest 15 Minuten lang fortzusetzen.

2. Ein Verfahren nach Anspruch 1, worin das Schwefeltrioxid mit einem anderen Gas verdünnt ist.

3. Ein Verfahren nach einem der Ansprüche 1 oder 2, worin das molare Verhältnis von Alkylbenzol zu Schwefeltrioxid in einem Bereich von 1 : 0,95 bis 1 : 1,1 liegt.

4. Ein Verfahren nach einem der Ansprüche 1, 2 oder 3, worin die Menge der Schwefelsäure in einem Bereich von 1 bis 5 Gewichtsprozent der Reaktionsmischung liegt.

5. Ein Verfahren nach einem der vorstehenden Ansprüche, worin es der Reaktion nach Inkorporierung der Schwefelsäure erlaubt ist, sich für einen Zeitraum in einem Bereich von 30 Minuten bis zu 8 Stunden fortzusetzen.

6. Ein Verfahren nach einem der vorstehenden Ansprüche, worin die Mischung auf einer Temperatur im Bereich von 30° bis 70°C nach darin erfolgter Inkorporierung der Schwefelsäure gehalten wird.

## Revendications

1. Un procédé de préparation d'alcoyl benzène sulfonates par mise en contact, comme corps en réaction, d'un alcoyl benzène avec l'anhydride sulfurique en utilisant un rapport molaire de l'alcoyl benzène à l'anhydride sulfurique compris entre 1:0,9 et 1:1,3, caractérisé en ce qu'on incorpore de l'acide sulfurique dans le mélange réactionnel après que les corps en réaction ont été mis en contact, la quantité d'acide sulfurique n'étant pas supérieure à 10 % en poids du mélange réactionnel, et qu'on laisse ensuite la réaction se poursuivre dans le mélange pendant au moins 15 minutes.

2. Un procédé selon la revendication 1, dans lequel l'anhydride sulfurique est dilué avec un autre gaz.

3. Un procédé selon la revendication 1 ou la revendication 2, dans lequel le rapport molaire de l'alcoyl benzène à l'anhydride sulfurique est compris dans un intervalle de 1:0,95 à 1:1,1.

4. Un procédé selon la revendication 1, la revendication 2 ou la revendication 3, dans lequel la quantité d'acide sulfurique est comprise dans un intervalle de 1 à 5 % en poids par rapport au mélange réactionnel.

5. Un procédé selon l'une quelconque des revendications précédentes, dans lequel, après l'incorporation de l'acide sulfurique, on laisse la réaction se poursuivre pendant un laps de temps compris dans un intervalle de 30 minutes à 8 heures.

6. Un procédé selon l'une quelconque des revendications précédentes, dans lequel le mélange est maintenu à une température comprise dans l'intervalle de 30 à 70°C après l'incorporation de l'acide sulfurique.
